# EUROPEAN PATENT APPLICATION

(11) **EP 3 392 264 A1**
(43) Date of publication of application: **24.10.2018**
(21) Application number: 16874961.2
(22) Date of filing: 16.12.2016
(51) Int. Cl.: C07K 7/06, C01G 5/00, C01G 7/00, H01B 1/22, H01B 5/06, B82B 1/00, B82B 3/00, B82Y 5/00, B82Y 15/00, B82Y 20/00, B82Y 30/00

(54) **MATERIAL COMPRISING OLIGOGLYCINE TECTOMERS AND NANOWIRES**

(30) Priority: 18.12.2015 ES 201531843
(71) Applicant: Consejo Superior De Investigaciones Científicas (CSIC), 28006 Madrid (ES); Universidad De Zaragoza, 50009 Zaragoza (ES); University of Surrey, Surrey GU2 7XH (GB)
(72) Inventor: MUÑOZ DE MIGUEL, Edgar Manuel, 50015 Zaragoza (ES); GARRIGA MATEO, Rosa, 50009 Zaragoza (ES); DALTON, Alan Brian, Falmer Brighton BN1 9RH (GB); JUREWICZ, Izabela, Guildford Surrey GU2 7XH (GB)
(74) Representative: Elzaburu S.L.P.
(86) International application number: PCT/ES2016/070904
(87) International publication number: WO 2017/103317

(57) **Abstract**

The invention relates to a material comprising oligoglycine tectomers and nanowires. This material is useful as an electrode, as a conductive and transparent hybrid material, and as a pH sensor, as well as in biomedical applications.

## Description

The present invention relates to a multifunctional hybrid material, comprising oligoglycine tectomers and nanowires (NWs). The present invention further relates to the procedure for obtaining such material and to its use as an electrode, as a conductive and transparent hybrid material, and as a pH sensor.

### PRIOR ART

In recent years there has been much research on the study of the synthesis, properties and applications of one-dimensional materials, and including nanowires (NWs) in particular. Their unique physical properties depending on their composition, together with their high aspect ratio and surface area, have led to the use of the NWs in multiple devices and applications, which may include electronic, electrochemical, photonic and biological applications, in energy storage devices, solar cells and sensors (see, for example, Materials Today 2006, 9, 18-27; Advanced Materials 2014, 26, 2137-2184). NWs of very varied compositions and properties have been described, e.g. metallic NWs (Au, Ag, Pt, Ni, among others), semiconducting NWs (Si, GaN, GaAs, ZnO, PbSe...), insulating NWs (TiO₂, SiO₂, among others) and organic NWs (e.g. of conductive polymers, Journal of the American Chemical Society 2005, 127, 496-497).

Silver nanowires (AgNWs) have a very high optical transmission but at the same time very low sheet resistance (Rs), which combined with their decreasing cost and current large-scale production methods, makes them perfect candidates in the area of transparent and flexible electronics (see, for example, Nano Letters 2012, 12, 3138-3144; ACS Nano 2009, 3, 1767-1774; ACS Applied Materials & Interfaces 2013, 5, 10165-10172; Materials Research Bulletin 2013, 48, 2944-2949; Nanoscale 2014, 6, 946-952). In addition, the synergy of AgNWs with two-dimensional materials such as graphene has been proven to provide additional functionalities to the electrodes, such as mechanical flexibility and improved electronic transport properties, which may make them commercially competitive against ITO (indium tin oxide) (Advanced Functional Materials 2014, 24, 7580-7587).

Gold nanowires (AuNWs) are very interesting for many applications due to their chemical stability, biocompatibility and high electrical conductivity, including use as transparent electrodes, (Procedia Engineering 2016, 141, 152-156), sensors, energy (solar cells) and biomedicine. The capacity of penetration of the gold wires in cells and tissues combined with the high electrical conductivity and handling capability under electric fields (Nat. Nanotechnol. 2011, 6, 57-64; Appl. Phys. Lett. 2004, 85, 4175) make AuNWs interesting for applications in biomedicine: diagnostic imaging (Chem. Commun., 2013,49, 11038-11040), biosensors and nanoinjection of exogenous agents (J. Mater. Sci. Tech. 2015, 31, 573-580). The functionalisation of AuNWs as a result of their surface modification is interesting for all these applications.

Oligoglycines able to assemble in two-dimensional systems, either spontaneously or in surface-assisted processes, have been described and synthesised, whose stability is based on the formation of hydrogen bonds (see, for example, ChemBioChem 2003, 4, 147-154; Nanotechnologies in Russia 2008, 3, 291 - 302; J. Org. Chem. 2014, 10, 1372-1382). These types of two-dimensional supramers are called tectomers. Tectomers are soluble in water as well as in an acid medium. The self-assembly and aggregation are favoured by shifting the pH of the medium to basic values. Tectomers have attracted attention in biomedicine because the adsorption of these peptide layers on the surface of virus blocks the adhesion of the virus to cells (see, for example, ChemBioChem, 2003, 4, 147-154; Glycoconjugate Journal 2004, 21, 471-478). On the other hand, the availability of tectomers able to form perfectly flat and rigid layers with a predefined thickness is promising for the design of new nanomaterials and as a platform for nanodevices (see, for example, J. Org. Chem. 2014, 10, 1372- 1382). Previous work has shown that tectomers can act as vehicles for pH-controlled loading and release of anticancer drugs and fluorescent substances (ACS Appl. Mater. Interfaces 2016, 8, 1913-1921).

Therefore, it would be desirable to have electrodes of AgNWs or AuNWs with improved physical-chemical properties, that do not require a high content of AgNWs or AuNWs due to their high cost.

### DESCRIPTION OF THE INVENTION

Thus, the present invention describes the synergistic effect observed between oligoglycine tectomers and NWs, particularly silver nanowires (AgNWs) or gold nanowires (AuNWs), to provide NW materials with improved physico-chemical properties.

In the case of AgNWs, tectomer coatings significantly reduce the sheet resistance (Rs) of the electrode, also acting as barriers to moisture, protection against high temperatures and maintaining electrode transparency. Rs reduction without increasing the amount of AgNWs is of interest due to their high cost and for the fabrication of transparent, flexible, wearable and cheap electrodes. In addition, oligoglycine allows reversible switching of the response to external stimuli such as the change of pH which makes it useful for the preparation of sensors.

Therefore, an aspect of the present invention relates to a material comprising oligoglycine tectomers and nanowires (NWs).

In another embodiment the invention relates to the material as defined above, wherein the NWs are silver nanowires (AgNWs).

In another embodiment the invention relates to the material as defined above, wherein the NWs are gold nanowires (AuNWs).

In another embodiment the invention relates to the material as defined above, wherein the oligoglycine tectomers are deposited on the NWs.

In another embodiment the invention relates to the material as defined above, characterised by being a substrate/NWs/tectomers multilayer system, and preferably a multilayer substrate/AgNWs/tectomers or substrate/AuNWs/tectomers system.

In these systems, the hybrid AgNW/tectomers or AuNW/tectomers remain attached to the substrate, although under certain conditions (for example, adjusting the pH) they can detach from the substrates, these hybrid materials themselves forming conductive and transparent film which can also be transferred to other substrates in liquid medium.

In another embodiment the invention relates to the material as defined above, characterised by being conductive and transparent film.

In another embodiment the invention relates to the material as defined above, characterised by being conductive and transparent film, which can be transferred to other substrates in liquid medium.

Another aspect of the present invention refers to the procedure for obtaining the material as mentioned above which comprises:
i) deposition, preferably by spray coating, of NWs on a substrate, preferably wherein the substrate is selected from glass, polyethylene terephthalate (PET) or polymethylmethacrylate (PMMA); and
ii) The deposition of solutions of oligoglycine tectomers on the films of NWs resulting from step (i).

For the deposition of AgNWs, dispersions of 0.1 mg/mL in isopropanol diluted from an original dispersion of 5 mg/mL were used. The average diameter and length of the AgNWs used are respectively 60 nanometers and 10 micrometers. Diluted dispersions are used for the fabrication of AgNW films, whose density on the substrates is controlled with the spray coated volume of the dispersions. The processes of spray coating of the dispersions of AgNWs are carried out at a pressure of 30 psi and a distance of 10 cm between the airbrush nozzle and substrate.

The density of AgNW films on the substrates is controlled by the spray coated volume when using dispersions of AgNWs of the same concentration, verifying the densities of AgNWs deposited by means of Rs and topography using atomic force microscopy (AFM).

Aqueous solutions of biantennary, triantennary and tetrantennary oligoglycine were used for the tectomer coatings. The deposition of the oligoglycine tectomers on the films of NWs was conducted by drop casting procedures, by immersion in oligoglycine tectomer solutions (dip coating), by dragging the dispersions on the substrates with a blade (doctor blade) or by centrifuging (spin coating). The density of AgNWs deposited by spray coating, and the concentration, volume and type of oligoglycine of the tectomer solutions define the composition of the coatings.

Aqueous solutions of biantennary, triantennary and tetrantennary oligoglycine were used for the tectomer coatings. The deposition of the oligoglycine tectomers on the films of NWs can be conducted by "drop casting" procedures, by immersion in oligoglycine tectomer solutions ("dip coating"), by dragging the dispersions on the substrates with a blade ("doctor blade") or by centrifuging ("spin coating"). The density of AgNWs deposited by spray coating, and the concentration, volume and type of oligoglycine of the tectomer solutions define the composition of the coatings.

In another embodiment the invention relates to the procedure as described above, wherein step (ii) is carried out by drop casting, dip coating, doctor blade or by spin coating.

Another aspect of the invention relates to the use of the material as defined previously as an electrode or as a transparent conducting component, and preferably as electrodes for transparent electronic and optoelectronic devices, touch screens, solar cells, sensors and biosensors.

Coating with oligoglycine tectomers also confers antimicrobial and antiviral features, protection against high temperatures (of up to 85 °C), as well as moisture barrier and self-cleaning/anti-fouling properties due to the increased hydrophobicity resulting from the tectomer/NWs synergy.

Thus, in another embodiment the invention relates to the use of the material as defined above, wherein the hybrid material has antimicrobial, antiviral, moisture barrier, self-cleaning and anti-fouling properties and those of protection against high temperatures, and preferably protection against temperatures of up to 85 °C.

Another aspect of the present invention relates to the use of the material as defined above, as conductive hybrid material characterised by having transparency values above 90%.

Another aspect of the present invention relates to the use of the material as previously defined as pH sensor.

Another aspect of the present invention relates to a device that comprises the material as defined above.

NW materials, particularly AgNWs or AuNWs, and oligoglycine would act as highly conductive components with a potential application in devices such as those mentioned in the preceding paragraph, and the oligoglycine may additionally provide improved hydrophobicity as well as new biofuncionalidades to the NWs, particularly AgNWs or AuNWs.

On the other hand, the NW/oligoglycine material, preferably AgNWs/oligoglycine or AuNWs/oligoglycine material, is used as a pH sensor since that the change in pH affects the degree of oligoglycine assembly, which can be monitored by microscopy studies.

The sheet resistance (Rs) is quantified in Ohm/sqr and is represented as Ohm/sqr.

Another aspect of the invention relates to the use of the material defined above for loading drugs and fluorescent substances.

Throughout the description, "oligoglycine tectomers" refers to aggregates of oligoglycine molecules, which self-assemble forming stable two-dimensional structures, both in solution and on surfaces. These structures are stabilised by the formation of hydrogen bonds in two dimensions between the glycines at the ends of the molecule (called antennas). Thus, these two-dimensional structures can be formed from biantennary, triantennary and tetrantennary oligoglycines. For the invention described herein this type of oligoglycine has been used, preferably biantennary oligoglycine.

"Nanowires" (NWs) refers to quasi-one-dimensional wires, having a diameter ranging from 1 nm to several tens of nanometers and typical lengths ranging from several hundreds of nanometer to several micrometres. The composition of the nanowires can be very varied, on which their chemical and physical properties depend. Examples of nanowires include among other conducting NWs (Au, Ag, Pt, Ni, among others), semiconducting NWs (Si, GaN, GaAs, ZnO, PbSe, among others), insulating NWs (Ti02, Si02, among others) and organic NWs. The invention described herein has preferably used conducting NWs (Au, Ag, Pt, Ni, among others), semiconducting NWs (Si, GaN, GaAs, ZnO, PbSe, among others), more preferably conducting NWs, and even more preferably silver nanowires (AgNWs). However, other types of NWs and quasi - one-dimensional materials may also be used in these systems if their interaction with the oligoglycine tectomers is favourable, either by their nature or composition, or by their functionalisation and/or charge, preferably carboxylated nanowires, since, due to the affinity to tectomers, with protonated amino groups, because of the negative charge surfaces (J. Biomater. Nanobiotech. 2011, 2, 91-97; ACS Appl. Mater. Interfaces 2016, 8, 1913-1921), a greater interaction at the interface between these functionalised nanowires and the tectomers is obtained. Thus, NWs of other compositions and properties as well as other one-dimensional materials such as nanotubes may also be used in these systems if their interaction with the oligoglycine tectomers is favourable, either due to composition or functionalisation.

Throughout the description, "hybrid system" refers to a system of two components: a layer of NWs, on which the oligoglycine tectomers are deposited, being primarily supported on a substrate.

Throughout the description and claims the word "comprises" and its variants are not intended to exclude other technical features, additives, components or steps. For those skilled in the art, other objects, advantages and characteristics of the invention will emerge partly from the description and partly from practice of the invention. The following examples and figures are provided by way of illustration and are not intended to limit the scope of the present invention.

### BRIEF DESCRIPTION OF THE FIGURES

- **FIG. 1.**: Transmittance (at 550 nm) vs Rs of AgNW films of different densities. This figure includes a representative atomic force microscopy (AFM) 3D topography image of an AgNW film of intermediate density.
- **FIG. 2**: Left: AFM topography image of individual layers of oligoglycine tectomers deposited on a glass substrate. Right: Height profile showing that individual tectomer layers are approximately 5.7 nm thick.
- **FIG. 3.**: The chemical structure of the biantennary oligoglycine, and the representation of its assembly in the form of tectomers deposited on a glass substrate.
- **FIG. 4**: (a) Sheet resistance (Rs) as a function of time of an AgNW medium-density electrode on which a solution of 0.5 mg/mL biantennary oligoglycine has been deposited, left to dry at room temperature. A diagram of the system used to measure the current vs voltage curves is included. (b) Change of Rs (in %) as a function of the oligoglycine concentration in the original aqueous solution.
- **FIG. 5**: 3D representation of AFM topography images of low density AgNW film deposited on glass substrates, showing (a) tectomer aggregates and (b) an individual tectomer layer deposited on a low density AgNW conducting film. AFM topography images and contact angle measurements of water on intermediate density AgNW films covered with tectomer layers from solutions of oligoglycine at a concentration (c, f) 0.01 mg/ml, (d, g) 0.5 mg/ml and (e, h) 1 mg/ml.
- **FIG. 6**: pH-dependent assembly and destruction tectomer layers in solution and on electrodes of AgNWs. Photograph of oligoglycine solutions at different pH values (b). Micrographs of transmission electron microscopy (TEM) of 1 mg/ml oligoglycine solution samples with at pH 2.2 (a) and pH 7.4 (c). AFM images that show how the topography of AgNW/tectomer hybrids deposited on glass substrates change when exposed to solutions of different pH values (d).
- **FIG. 7.**: Kinetics of bacteria growth (a) E. Coli and (b) salmonella on a glass substrate using as culture medium brain heart infusion *(BHI broth),* (▲) without and (•) in the presence of AgNW/tectomer.
- **FIG. 8**: Transmission electronic microscopy images (TEM) of AuNW/tectomer hybrids.

### EXAMPLES

Below the invention will be illustrated by means of tests performed by the inventors, which highlights the effectiveness of the product of the invention.

### Example 1: Preparation of the material

For the fabrication of the material, AgNWs have been used, which have high transparency and electrical conductivity, and which have also proven to be exceptional materials for multiple applications ranging from capacitive touch sensors to multifunctional wearable sensors (see, for example, Advanced Functional Materials 2014, 24, 7580-7587; Nanoscale 2014, 6, 2345-2352; ACS Nano 2014, 8, 5154-5163). The AgNWs are deposited by spray coating on glass substrates, forming layers of different (and controlled) densities. AgNW deposition on substrates of polyethylene terephthalate (PET) and (poly(methyl methacrylate), PMMA) was also performed, which gives the material high transparency and flexibility.

Aqueous solutions of biantennary, triantennary and tetrantennary oligoglycine were used for the tectomer coatings. The deposition of the oligoglycine tectomers on the films of NWs was conducted by drop casting procedures, by immersion in oligoglycine tectomer solutions (dip coating), by dragging the dispersions on the substrates with a blade (doctor blade) or by centrifugation (spin coating).
- Drop casting: The tectomer solutions were drop casted on AgNW film and left to dry at room temperature for 3 hours, or until the water had evaporated completely.
- Dip coating: Solutions were deposited by dip coating of the substrate for 10 seconds, after which the substrate is removed from the solution at a speed of 1 mm/min. Once the process has been completed, the substrate with its coating is left dry in an upright position for 15 minutes.
- Doctor blade: Is a suitable procedure for obtaining large tectomer coatings. 2 mL of tectomer solution were deposited at the end of a substrate and were then spread evenly over the substrate. In these experiments the blade height used was 50 micrometers; it can be adjusted in each case.
- Spin-coating: the tectomer solutions were deposited on the spray coated AgNW films and spun at 2000 rpm for 10 seconds.

The density of AgNWs deposited by spray coating, and the concentration, volume and type of oligoglycine of the tectomer solutions define the composition of the coatings.

The fabrication of the material comprising AuNWs takes place by a procedure analogous to the above with the corresponding starting materials, i.e. with AuNWs instead of AgNWs.

### Example 2: Transmittance (T) vs sheet resistance (Rs)

Thus, transmittance (T) vs Rs curves were obtained. The choice of the deposition system was justified by the fact that spray coating is industrially scalable and can produce films with high transparency on large substrates. Three types of AgNW electrodes, Rs = 50 Ohm / sq, 1 kOhm / sq, and 1 MOhm / sq, were prepared depending on the density (high, intermediate and low, respectively) of AgNWs in the AgNW films.

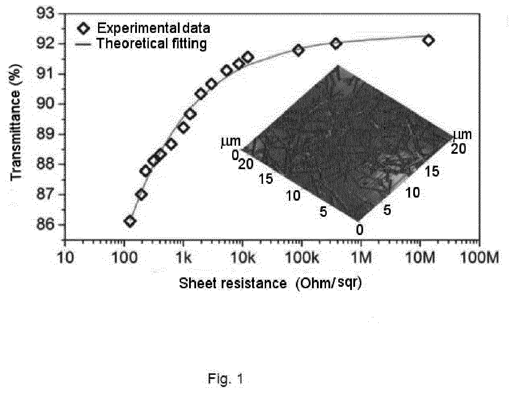

As shown in Fig. 1, the transmittance is greater than 92% for low densities of AgNWs and gradually decreases as AgNWs are added.
The AFM 3D topography image of the intermediate density AgNW film included in Fig. 1 provides values of Rs∼10 kOhm / sqr as well as transparency values of 91%, with 2-3 layers of AgNWs evenly distributed on the substrate.

### Example 3: Morphology of oligoglycine tectomer layers on glass

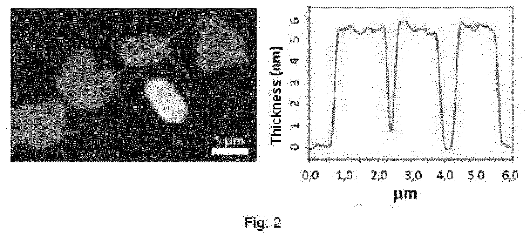

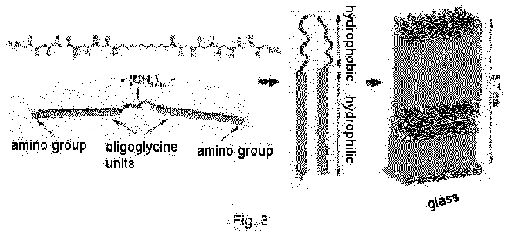

AFM has been used to study the morphology of the oligoglycine tectomer layers deposited on a glass substrate (Fig. 2). AFM characterisation shows that the oligoglycine molecules are assembled forming two-dimensional systems or platelets whose sizes are in the micrometer range (Fig. 2 left), each individual platelet being approximately 5.7 nm thick (Fig. 2 right). Each tectomer platelet consists of a coplanar stacking of biantennary oligoglycine molecules, facing each other such that its hydrophobic component is always exposed on the surface, as shown in the diagram of Fig. 3. Tectomer layers are atomically-smooth, highly uniform, and present no structural defects.

### Example 4: Current and voltage as a function of time

Oligoglycine solutions have been deposited on AgNWs, obtaining for each sample current vs voltage (I-V) curves as a function of time. I-V curves of the AgNW films and AgNWs/oligoglycine hybrid systems showed an excellent ohmic behaviour, regardless of the density of AgNWs and of concentration of oligoglycine used.

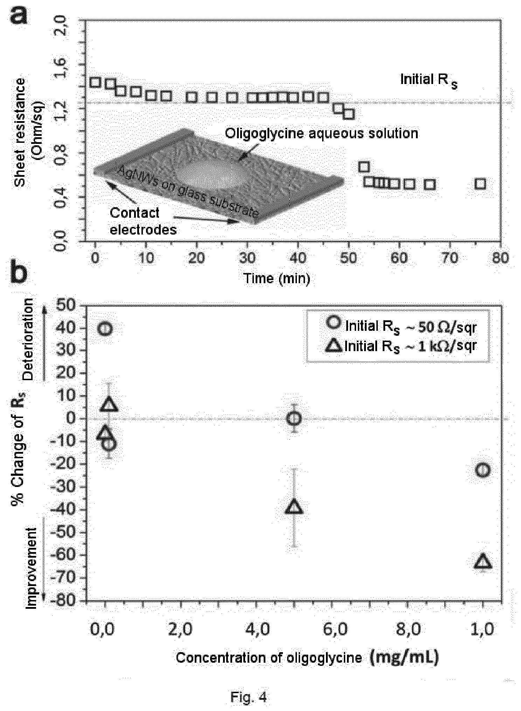

The diagram of the system used to make these measurements is shown in Fig. 4a. In films with an intermediate content in AgNWs there is a sharp decrease (between 50-70%) of the initial value of Rs when oligoglycine solutions of 0.5 and 1.0 mg/mL were deposited (Fig. 4.b). This decrease in the value of Rs is due to the action of the tectomer layers deposited. The concentration in oligoglycine increases during the water evaporation process of the solutions deposited, and the tectomer layers and aggregates mechanically press the AgNWs, increasing the contact between individual AgNWs. This increase in conductivity is achieved while maintaining at all times transparency of baseline electrodes (transparency values above 90% with transparency changes as low as 1% with respect of the starting AgNW electrodes have been measured in the AgNW/oligoglycine materials of this study). In this regard, tectomer layers would have a behaviour similar to that described for graphene, which similarly mechanically press the AgNWs on which they are deposited (see, for example, Advanced Functional Materials 2014, 24, 7580-7587; ACS Applied Materials & Interfaces 2013, 5, 11756-11761; Sci. Rep. 2013, 3, 1112).

The effective interaction between the oligoglycine and the AgNWs in these systems and that produces the synergistic effects described herein also entails significant changes observed by X-ray photoelectron spectroscopy (XPS) in the peaks corresponding to Ag3d, N1s and O1s as a result of the deposition of the tectomer solutions on electrodes of AgNWs. This interaction between the tectomers and the NWs can be modulated if the NWs are functionalised. For example, the electrostatic interactions between functional groups of functionalised NWs and protonated terminal amino groups of the tectomers change the degree of interaction between the two components and the structure of the hybrid material.

### Example 5: Hydrophobicity

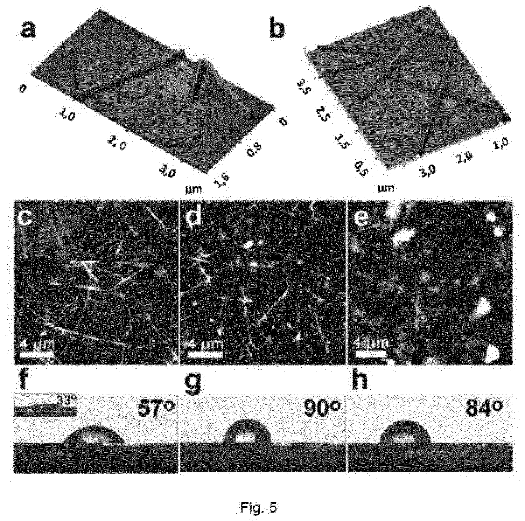

Due to the unique way in which the biantennary oligoglycine self-assembles in the form of tectomers in these experiments, its hydrophobic part is always exposed to the air. As a result, the water contact angle studies have been conducted to determine the hydrophobicity of tectomer-coated AgNW electrodes. These studies show a significant hydrophobicity increase (-90°) of the AgNW electrodes coated with tectomers from solutions of 0.5 and 1.0 mg/mL of biantennary oligoglycine (Fig. 5). This result indicates that the tectomer coatings act as an effective barrier to moisture, giving the system self-cleaning and anti-fouling functions. When the water drop is deposited on the AgNW electrode without tectomer coating, conversely, the water contact angle decreases from 33° to 11°, which indicates that the water penetrates into the unprotected conducting AgNW film.

### Example 6: AgNW/oligoglycine hybrids as pH sensors

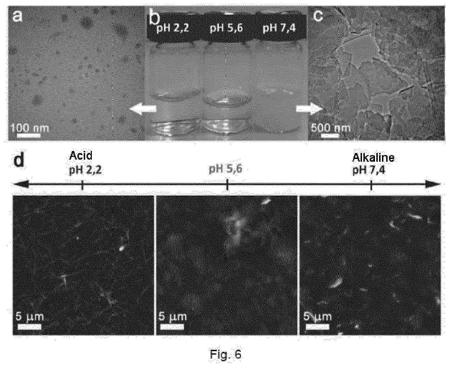

On the other hand, when the pH of an oligoglycine solution changes to acidic or basic regions, the oligoglycine assembly may take place or otherwise be destroyed (Fig. 6 a-c), since the change in pH can make the terminal amino groups of the oligoglycine neutral or charged (Beilstein Journal of Organic Chemistry 2014, 10, 1372-1382). The self-assembling or destruction of the tectomer layers is completely reversible and can be carried out repeatedly. These pH-dependent structural changes of oligoglycine can be observed also in hybrid electrodes of AgNWs/oligoglycine (Fig. 6.d) and can therefore be considered as pH sensors.

Oligoglycine coatings provide new biofuncionalidades to AgNW electrodes. As oligoglycines have the ability to physically or chemically immobilise viruses and bacteria (Russ J Bioorg Chem 2010, 36, 574-580), their hybridisation with AgNWs gives rise to new functionalities that make them useful as biomedical materials. In this regard, hybrids of AgNWs and two-dimensional peptide systems give rise to the formation of sophisticated nanostructures with smart response to the environment. In addition, the interaction of viruses and other analytes in these hybrid systems can be controlled through the functionalisation of terminal amino groups of oligoglycines, which makes them useful as biosensors or for the immobilisation of bacteria and viruses. The adhesion of viruses, bacteria, or other analytes to these films and electrodes of tectomer and AgNW hybrids can be removed by varying the pH of the environment, such that at low pH values the oligoglycine assemblies are destroyed, thus removing the materials attached to them.

### Example 7: Use of tectomer coatings as protection for AgNWs under extreme environmental conditions.

The performance of the transparent AgNW electrodes largely depends on the degradation and deterioration of the AgNWs themselves (Small 2014, 10, 4171-4181). Under environmental conditions, AgNWs undergo oxidation and sulfurization processes, so that it is highly important to protect the AgNW electrodes against harsh environmental conditions, such as for example humidity, exposure to oxygen and ozone as well as other gaseous molecules containing sulphur. Accordingly, different coatings that protect the nanowires against deterioration in these conditions have been developed, either by coating the individual nanowires or systems consisting of a set of nanowires (ACS Appl. Mater. Interfaces 2012, 4, 6410-6414; Nanoscale 2014, 6, 4812-4818; ACS Appl. Mater. Interfaces 2015, 7, 23297-23304; J. Nanoparticle Res. 2012, 14, 1-9; Appl. Phys. Lett. 2011, 99, 183307). In this case, a tectomer solution was drop casted on AgNW films until they were fully covered, and left to dry at room temperature. AgNW electrodes with and without tectomer coating were exposed to high temperature (85° C) and high relative humidity (85%) atmosphere. After 2 hours of exposure to these extreme environmental conditions, sheet resistance of the AgNW electrodes was measured, noting that it increased only by 10-14% with tectomer coating, while a 200 % increase in sheet resistance took place for the uncoated AgNW electrodes. Therefore, these results show that tectomer coatings provide protection to AgNW systems under harsh environmental conditions.

### Example 8: AgNWs coated with tectomers exhibit antimicrobial activity.

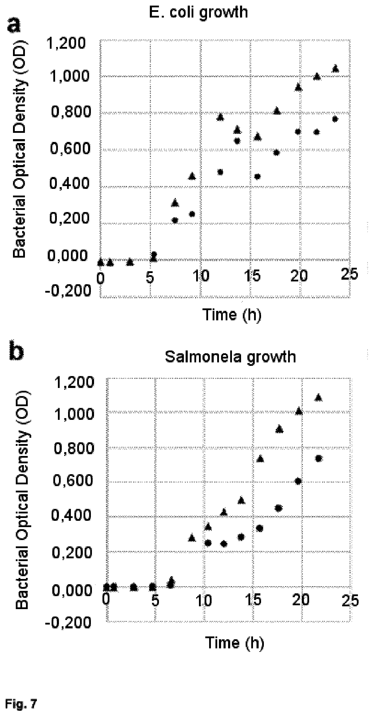

Studies on the kinetics of E. coli and salmonella growth using as culture medium a brain-heart infusion (BHI broth), in AgNW/tectomer materials deposited on glass, show that bacterial growth is significantly inhibited in these compared with the substrate used without coating (Fig. 7). These results show that coatings with tectomers protect not only the AgNWs against deterioration produced by the environment (example 7), but it also preserves the antimicrobial activity of the AgNWs (Mater. Sci. Eng. C 2017, 70,1011-1017). The adhesive action of tectomers on bacteria (Colloids Surf. A 2014, 460, 130-136) and viruses (ChemBioChem 2003, 4, 147- 154; Glycoconjugate J. 2004,1, 471-478), favours the contact with the AgNWs, and therefore their antimicrobial activity. Indeed, previous work has shown that tectomers can be loaded with drugs (ACS Appl. Mater. Interfaces, 2016, 8, 1913-1921), so the effectiveness of the antimicrobial activity of these materials can be increased, while preserving the properties of the AgNWs.

### Example 9: Interaction of tectomers with gold nanowires.

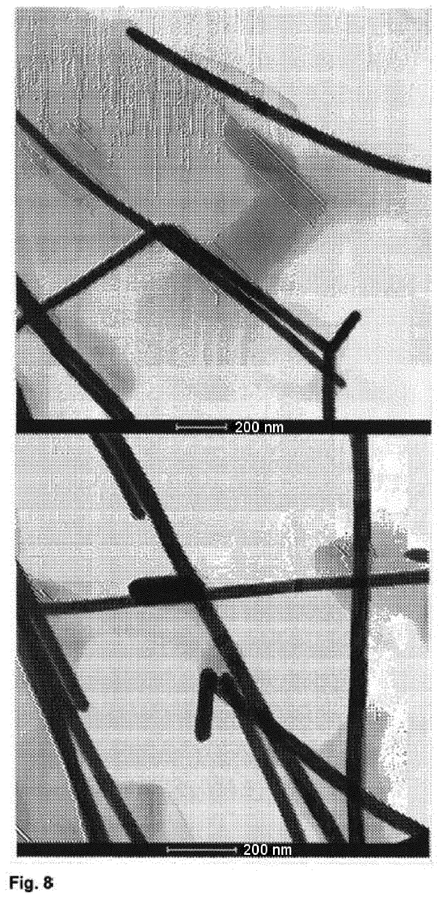

AuNW/tectomer hybrids have been prepared. The AuNWs used had an average diameter of 30 nm and lengths of 4.5 µm. TEM characterisation of the aqueous dispersions resulting from mixing 0.5 mg·mL-1 of biantennary oligoglycine and 0.05 mg·mL-1 of AuNWs is shown in Fig. 8, in which there is an extensive coating of AuNWs by the oligoglycine tectomers.

The functionalisation of AuNWs as a result of surface modification with tectomers is promising for applications in electronics, sensors, energy (solar cells) and biomedicine. Thus, AuNW/tectomer hybrids can combine the features of biomedical interest of both the AuNWs and the tectomers, which can be loaded with drugs and fluorescent substances (ACS Appl. Mater. Interfaces 2016, 8, 1913-1921).

In addition, the procedures described herein can be extended to functionalised nanowires, such as for example carboxylated nanowires.

## Claims

1. Material comprising oligoglycine tectomers and nanowires (NWs).

2. The material according to claim 1, wherein the NWs are silver nanowires (AgNWs).

3. The material according to claim 1, wherein the NWs are gold nanowires (AuNWs).

4. Material according to any of claims 1 to 3, wherein the oligoglycine tectomers are deposited on the NWs.

5. Method for obtaining the material according to any of claims 1 to 4, comprising:
i) the deposition, preferably by spray coating, of NWs on a substrate, preferably wherein the substrate is selected from glass, polyethylene terephthalate (PET) or polymethylmethacrylate (PMMA); and
ii) the deposition of solutions of oligoglycine tectomers on the films of NWs resulting from step (i).

6. The procedure according to claim 5, wherein step (ii) is carried out by drop casting, dip coating, doctor blade or by spin coating.

7. Use of the material according to any of claims 1 to 4, as an electrode or as a transparent conducting component.

8. Use according to claim 7, as an electrode for transparent electronic and optoelectronic devices, touch screens, solar cells, sensors and biosensors.

9. Use according to claim 7, wherein the hybrid material has antimicrobial, antiviral, moisture barrier and protection properties, against high temperatures and self-cleaning agent anti-fouling properties.

10. Use according to claim 9, wherein the hybrid material has properties for the protection against temperatures of up to 85°C.

11. Use of the material according to any of claims 1 to 4, as a conducting hybrid material **characterised by** having transparency values above 90%.

12. Use of the material according to any of claims 1 to 4, as pH sensor.

13. Use of the material according to any of claims 1 to 4, for loading drugs and fluorescent substances.

14. Device comprising the material according to any of claims 1 to 4.
